# FASCICULE DE BREVET EUROPEEN

(11) **EP 2 310 140 B1**
(45) Date de publication et mention de la délivrance du brevet: **22.05.2013**
(21) Numéro de dépôt: 09766074.0
(22) Date de dépôt: 16.06.2009
(51) Int. Cl.: B65D 83/18, B05B 11/00, B65D 83/38, B65D 83/20, A61M 15/00

(54) **DISPOSITIF DE DISTRIBUTION DE PRODUIT FLUIDE**
AUFTRAGVORRICHTUNG EINES FLUIDS
DISPENSING DEVICE FOR A FLUID PRODUCT

(30) Priorité: 17.06.2008 FR 0853996
(43) Date de publication de la demande: 20.04.2011
(73) Titulaire: Aptar France SAS, 27110 Le Neubourg (FR)
(72) Inventeur: PETIT, Ludovic, F-27110 Vitot (FR); POTTIER, Xavier, F-27680 Saint Samson de la Roque (FR)
(74) Mandataire: CAPRI
(86) Numéro de dépôt international: PCT/FR2009/051134
(87) Numéro de publication internationale: WO 2009/153512

(56) Documents cités:
- EP-A- 1 170 061
- WO-A-01/03851
- WO-A-02/20168
- WO-A-03/095007
- WO-A-2005/075105

## Description

La présente invention concerne un dispositif de distribution de produit fluide, et plus particulièrement un dispositif de pulvérisation nasale d'un produit pharmaceutique.

Les dispositifs de distribution de produit fluide sont bien connus dans l'état de la technique. Ils comportent généralement un réservoir contenant le produit fluide sur lequel est assemblé un organe de distribution, par exemple une pompe ou une valve, qui est généralement actionné au moyen d'une tête de distribution pour distribuer de manière sélective le produit contenu à l'intérieur dudit réservoir. La tête de distribution comporte un orifice de distribution à travers lequel le produit va être pulvérisé, par exemple dans le nez de l'utilisateur dans le cas d'un dispositif de pulvérisation nasale. De nombreux dispositifs de ce type sont actionnés manuellement par l'utilisateur en déplaçant axialement l'un contre l'autre le réservoir et la tête de distribution, ce qui a pour effet d'actionner l'organe de distribution. Ce type de dispositif présente toutefois des inconvénients, notamment lorsque le dispositif est du type à pulvérisation nasale, car la force axiale exercée par l'utilisateur pour actionner le dispositif induit un risque de déplacement de la tête de distribution à l'intérieur de la narine de l'utilisateur, avec des risques de blessure et/ou de distribution incomplète ou inadéquate du produit au moment de l'actionnement. Pour remédier à ce problème, des dispositifs d'actionnement latéral ont été proposés, comportant généralement un levier monté pivotant sur un corps et dont une partie interne est adaptée à coopérer avec l'un parmi la tête de distribution ou le réservoir pour déplacer cet élément contre l'autre et ainsi actionner l'organe de distribution. Ces dispositifs sont toutefois généralement assez complexes, et comportent un grand nombre de parties constitutives, ce qui rend la fabrication et l'assemblage relativement coûteux. De même l'interface entre le système d'actionnement latéral et l'organe de distribution pour transférer le déplacement radial du système d'actionnement en déplacement axial de l'organe de distribution, pour réaliser l'actionnement, est souvent complexe et source de dysfonctionnements. Les documents WO 01/03851, WO 03/095007, WO 02/20168, WO 2005/075105 et EP-1 170 061 décrivent des dispositifs de l'art antérieur.

Le document WO 01/03851 décrit un dispositif selon le préambule de la revendication 1.

La présente invention a pour but de fournir un dispositif de distribution de produit fluide qui ne reproduit pas les inconvénients susmentionnés.

La présente invention a aussi pour but de fournir un dispositif de distribution de produit fluide, notamment de pulvérisation nasale, qui garantit un actionnement sûr et fiable du dispositif à chaque actionnement, sans risque de blessure pour l'utilisateur.

Plus particulièrement, la présente invention a pour but de fournir un dispositif de distribution de produit fluide qui soit simple et peu coûteux à fabriquer et à assembler.

La présente invention a donc pour objet un dispositif de distribution de produit fluide, comportant un corps, un réservoir, un organe de distribution, tel que une pompe ou une valve, monté sur le réservoir, un système d'actionnement latéral solidaire dudit corps, le dispositif comportant une bague montée sur ou solidaire du réservoir, ladite bague comportant au moins une projection radiale adaptée à coopérer avec un élément mobile dudit système d'actionnement latéral, ladite au moins une projection étant chanfreinée avec une surface d'extrémité radiale inclinée.

Avantageusement, ladite bague comporte deux projections radiales coaxiales et symétriques s'étendant respectivement de chaque côté de ladite bague.

Avantageusement, ladite au moins une projection a une section arrondie, notamment circulaire.

Avantageusement, ledit élément mobile dudit système d'actionnement latéral comporte un oeillet pour chaque projection radiale de la bague, ledit oeillet entourant ladite projection radiale.

Avantageusement, ledit oeillet a un profil interne arrondi, notamment oblong.

Avantageusement, l'organe de distribution est une pompe comportant un piston coulissant dans un corps de pompe, ladite bague comportant un manchon axial dont une première extrémité axiale forme une virole définissant la position de repos du piston, et dont une seconde extrémité axiale est réalisée sur une partie tubulaire de guidage adaptée à guider le piston lors de son coulissement.

Avantageusement, un poussoir est assemblé sur ladite pompe, ledit poussoir comportant une partie venant s'assembler à l'intérieur de ladite seconde extrémité axiale.

Avantageusement, ledit manchon axial s'étend jusqu'à l'extrémité axiale dudit piston.

Avantageusement, la seconde extrémité axiale est chanfreinée pour faciliter l'assemblage dudit poussoir.

Avantageusement, ladite bague est la bague de fixation dudit organe de distribution sur ledit réservoir.

Ces caractéristiques et avantages et d'autres de la présente invention apparaîtront plus clairement au cours de la description détaillée suivante de plusieurs modes de réalisation de l'invention, faite en référence aux dessins joints, donnés à titre d'exemples non limitatifs, et sur lesquels :
- la figure 1 est une vue schématique en perspective d'une bague selon un mode de réalisation de la présente invention ;
- la figure 2 est une vue similaire à celle de la figure 1, montrant une autre variante de réalisation de la bague de l'invention ;
- les figures 3 et 4 sont des vues schématiques d'un dispositif de distribution de produit fluide selon un mode de réalisation avantageux de la présente invention, incorporant une bague telle que représentée sur les figures 1 ou 2, respectivement en position de repos et en position d'actionnement ;
- la figure 5 est une vue schématique partielle en section d'un autre mode de réalisation de l'invention ; et
- la figure 6 est une vue similaire à celle de la figure 5, montrant encore une autre variante de réalisation ;

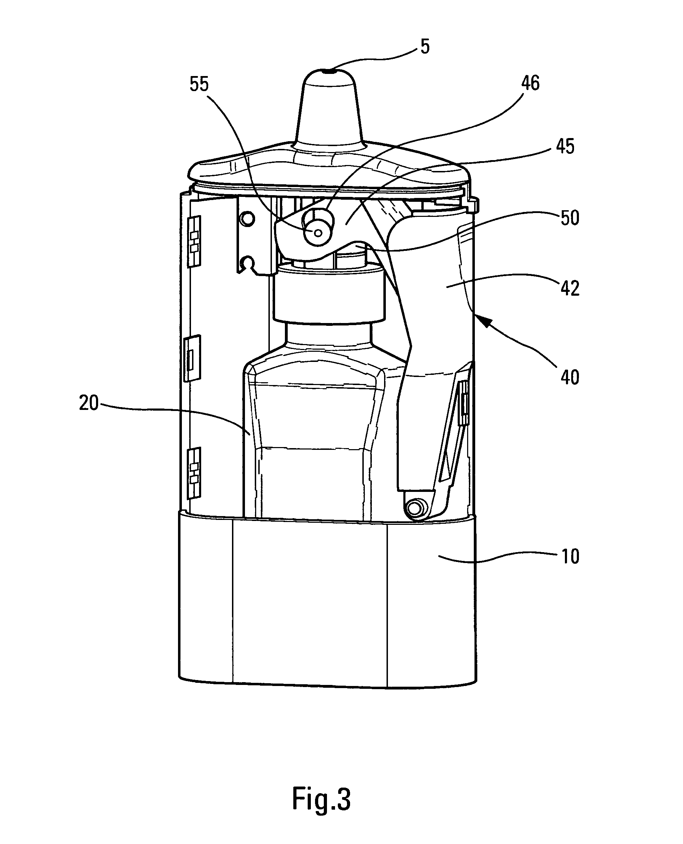

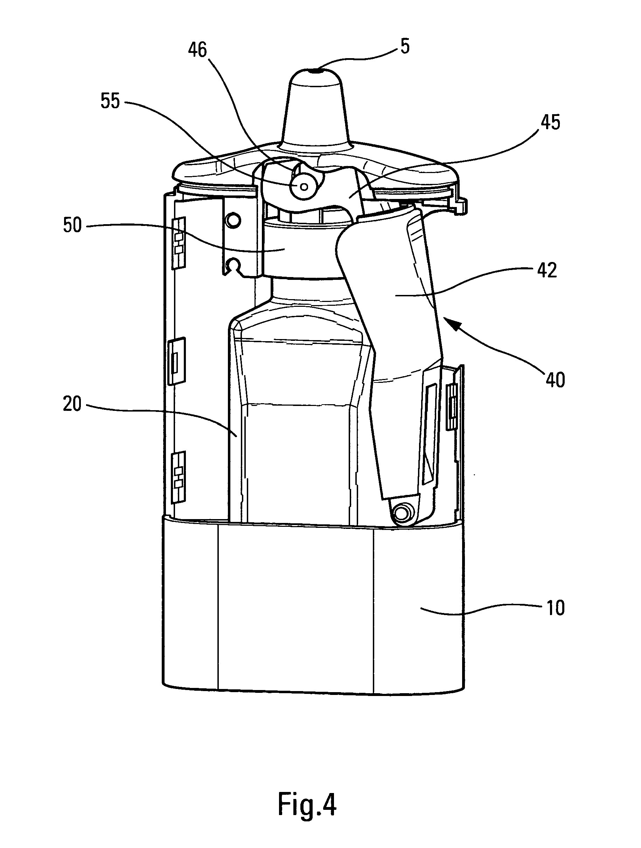

Selon un premier aspect de l'invention, un dispositif de distribution de produit fluide comporte un corps 10 représenté notamment sur les figures 3 et 4. Ce corps 10 incorpore avantageusement un orifice de distribution 5. A l'intérieur de ce corps est disposé un réservoir 20 contenant un produit fluide à distribuer. Un organe de distribution 30 (non visible sur les figures 3 et 4) est assemblé sur ledit réservoir, en particulier au moyen d'une bague de fixation de manière connue. L'unité formée du réservoir 20 et de l'organe de distribution 30 est capable de se déplacer axialement à l'intérieur du corps 10 lors de l'actionnement pour distribuer une dose de produit à travers l'orifice de distribution 5. Pour réaliser cet actionnement, le corps 10 comporte un système d'actionnement latéral 40, qui est solidaire du corps, et qui peut comporter notamment un levier 42 monté pivotant sur le corps 10 ainsi qu'un élément mobile 45 monté pivotant à l'intérieur dudit levier 42 et relié à l'unité formée par le réservoir 20 et l'organe de distribution 30. Selon l'invention, cet élément mobile 45 du système d'actionnement latéral coopère avec une bague 50 qui est montée sur ou solidaire du réservoir 20, ladite bague 50 comportant au moins une projection radiale 55 adaptée à coopérer avec ledit élément mobile 45.

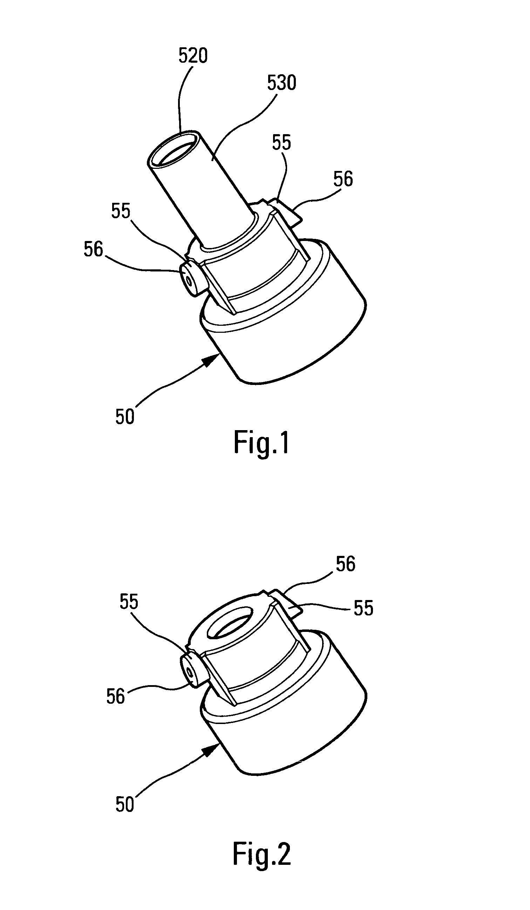

Les figures 1 et 2 représentent deux variantes de réalisation d'une telle bague selon l'invention. Cette bague 50 est de préférence l'organe qui fixe l'organe de distribution 30 sur le réservoir 20. Toutefois, on pourrait aussi envisager que cette bague soit une bague rapportée par-dessus l'élément de fixation qui sert à fixer l'organe de distribution sur le réservoir. De préférence, la bague 50 comporte deux projections radiales 55 qui sont co-axiales et qui s'étendent de manière symétrique respectivement de chaque côté de ladite bague 50. Avantageusement, les projections 55 ont une section transversale arrondie, notamment circulaire. Ceci est notamment souhaitable lorsque l'élément mobile 45 vient s'assembler autour de ladite projection (ou desdites projections) 55, auquel cas ledit élément mobile 45 comporte un oeillet 46 pour chaque projection radiale 55, ledit oeillet 46 entourant ladite projection radiale 55 comme visible notamment sur les figures 3 et 4. De préférence, ledit oeillet 46 a un contour ou profil interne arrondi, notamment oblong, qui coopère avec la forme extérieure arrondie de la projection 55, permettant ainsi un pivotement de l'élément mobile 45 autour de la projection 55 avec un minimum de frottement et donc un minimum d'impact sur la résistance à l'actionnement du dispositif. Eventuellement, l'oeillet 46 peut être de dimension supérieure à celle de la projection 55, comme visible sur les figures 3 et 4. En variante, on pourrait aussi envisager un oeillet dont les dimensions seraient environ identiques à celles de la projection 55. Selon l'invention, les projections 55 sont chanfreinées avec une surface d'extrémité radiale 56 inclinée, et notamment inclinée en direction de l'orifice de distribution 5. Cette mise en oeuvre simplifie l'assemblage du système d'actionnement latéral, et notamment l'assemblage de l'oeillet 46 autour de la projection 55. En venant emmancher ledit oeillet 46 par au-dessus, la surface d'extrémité radiale inclinée 56 de la projection 55 déforme l'élément mobile 45 vers l'extérieur, celui-ci venant ensuite s'encliqueter en dessous de ladite projection 55. Avec une telle mise en oeuvre, il n'y a aucun risque que l'élément mobile 45 se désolidarise de la projection 55 lors des futures utilisations du système.

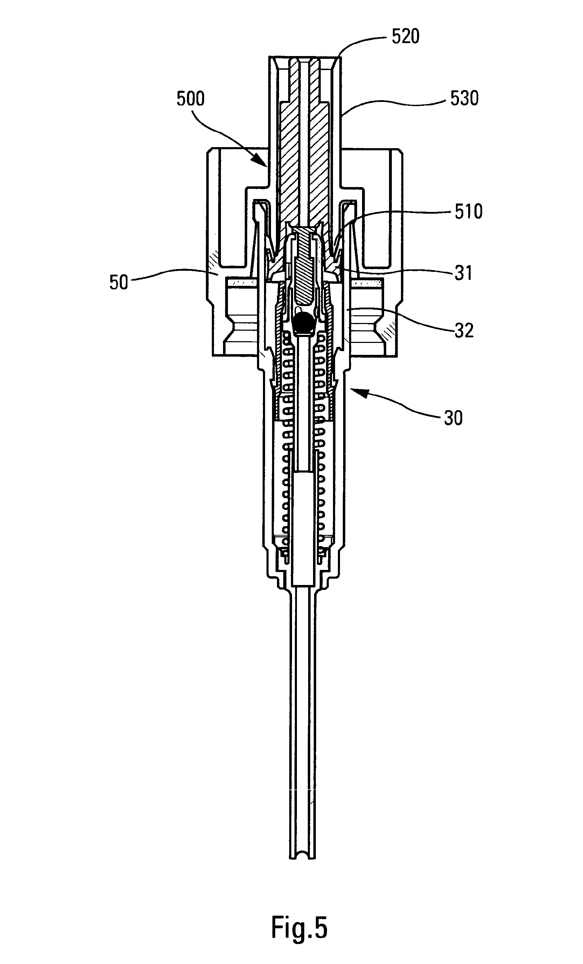

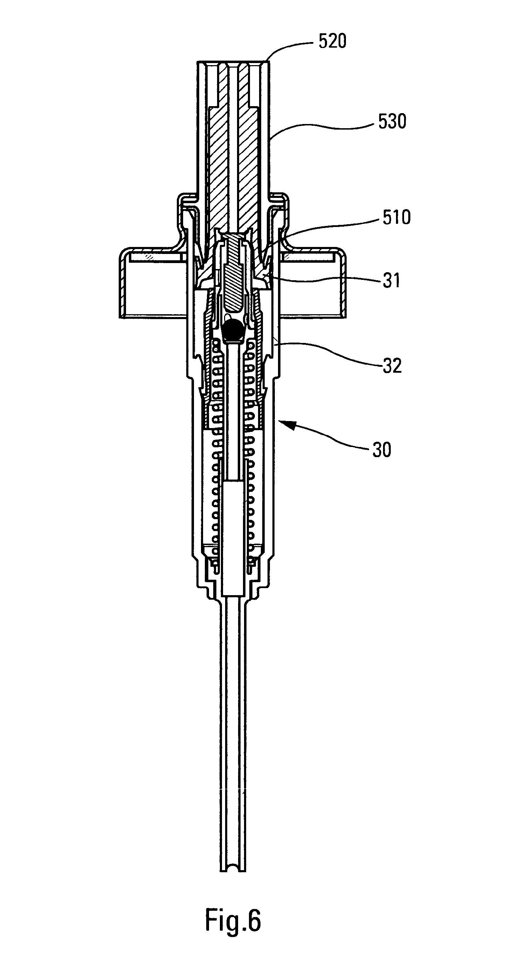

Lorsque l'organe de distribution 30 est une pompe, comme représenté sur les figures 5 et 6, comportant un corps de pompe 32 dans lequel coulisse un piston 31, ladite bague 50 forme avantageusement une virole qui définit la position de repos dudit piston 31, comme visible sur les figures 5 et 6. De préférence, la bague 50 comporte alors un manchon axial 500 dont une première extrémité axiale 510 forme ladite virole, en l'occurrence l'extrémité axiale inférieure dans la position droite représentée sur les figures 5 et 6. Avantageusement l'autre extrémité axiale 520 est réalisée sur une partie tubulaire de guidage 530 qui est adaptée à guider le piston 31 lors de son coulissement. La figure 1 montre une telle partie tubulaire de guidage 530, de même que les figures 5 et 6.

Eventuellement, un poussoir (non représenté), peut être assemblé sur la pompe 30 représentée sur les figures 5 et 6, auquel cas le poussoir peut comporter une partie qui vient s'assembler à l'intérieur de ladite seconde extrémité axiale 520. Cette seconde extrémité axiale 520 est alors de préférence chanfreinée pour faciliter l'assemblage dudit poussoir.

Comme visible sur les figures 5 et 6, le manchon axial 530 s'étend sensiblement jusqu'à l'extrémité axiale supérieure du piston 31 dans la position droite représentée sur les figures 5 et 6. Ceci facilite l'assemblage du sous-ensemble pompe-réservoir dans le système d'actionnement latéral 40.

Il est à noter que sur la figure 5, les projections radiales 55 ne sont pas représentées sur la bague de fixation 50. En effet, selon un autre aspect de l'invention, il est aussi envisageable d'utiliser une bague de fixation 50 qui sert à fixer une pompe 30 à un réservoir 20 et qui incorpore une telle partie tubulaire de guidage du piston, indépendamment du système d'actionnement, et donc aussi sans système d'actionnement latéral. Ceci permet d'éviter tout risque de « tiltage » de la pompe en cas d'actionnement par l'utilisateur qui ne serait pas axial, limitant ainsi les risques de blocage de la pompe, de fuites ou de distribution de dose incomplète susceptibles de survenir en cas de tiltage.

Bien entendu, lorsque utilisé avec un système d'actionnement latéral, la bague 50 de la figure 5 comporterait avantageusement également les deux projections latérales 55 décrites précédemment.

La figure 6 représente une autre variante de réalisation, dans laquelle la partie tubulaire de guidage n'est pas réalisée sur la bague qui sert à fixer la pompe sur le réservoir, mais uniquement sur la virole, c'est-à-dire sur l'élément qui définit la position de repos du piston 31. A nouveau, une telle virole pourrait bien entendu être utilisée indépendamment du système d'actionnement, que celui-ci soit axial ou latéral.

Bien que l'invention ait été décrite en référence à diverses variantes de réalisation de celle-ci, il est entendu qu'un homme du métier peut y apporter toutes modifications sans sortir du cadre de la présente invention tel que défini par les revendications annexées.

## Revendications

1. Dispositif de distribution de produit fluide, comportant un corps (10), un réservoir (20), un organe de distribution (30), tel que une pompe ou une valve, monté sur le réservoir (20), un système d' actionnement latéral (40) solidaire dudit corps (10), le dispositif comportant une bague (50) montée sur ou solidaire du réservoir (20), ladite bague (50) comportant au moins une projection radiale (55) adaptée à coopérer avec un élément mobile (45) dudit système d'actionnement latéral (40), **caractérisé en ce que** ladite au moins une projection (55) est chanfreinée avec une surface d'extrémité radiale (56) inclinée.

2. Dispositif selon la revendication 1, dans lequel ladite bague (50) comporte deux projections radiales (55) coaxiales et symétriques s'étendant respectivement de chaque côté de ladite bague (50).

3. Dispositif selon la revendication 1 ou 2, dans lequel ladite au moins une projection (55) a une section arrondie, notamment circulaire.

4. Dispositif selon l'une quelconque des revendications précédentes, dans lequel ledit élément mobile (45) dudit système d'actionnement latéral (40) comporte un oeillet (46) pour chaque projection radiale (55) de la bague (50), ledit oeillet (46) entourant ladite projection radiale (55).

5. Dispositif selon la revendication 4, dans lequel ledit oeillet (46) a un contour interne arrondi, notamment oblong.

6. Dispositif selon l'une quelconque des revendications précédentes, dans lequel l'organe de distribution (30) est une pompe comportant un piston (31) coulissant dans un corps de pompe (32), ladite bague (50) comportant un manchon axial (500) dont une première extrémité axiale (51) forme une virole définissant la position de repos du piston (31), et dont une seconde extrémité axiale (520) est réalisée sur une partie tubulaire de guidage (530) adaptée à guider le piston (31) lors de son coulissement.

7. Dispositif selon la revendication 6, dans lequel un poussoir est assemblé sur ladite pompe (30), ledit poussoir comportant une partie venant s'assembler à l'intérieur de ladite seconde extrémité axiale (520).

8. Dispositif selon la revendication 7, dans lequel ledit manchon axial (530) s'étend jusqu'à l'extrémité axiale dudit piston (31).

9. Dispositif selon la revendication 7 ou 8, dans lequel la seconde extrémité axiale (520) est chanfreinée pour faciliter l'assemblage dudit poussoir.

10. Dispositif selon l'une quelconque des revendications précédentes, dans lequel ladite bague (50) est la bague de fixation dudit organe de distribution (30) sur ledit réservoir (20).

## Patentansprüche

1. Ausgabevorrichtung für ein fluides Produkt, aufweisend einen Körper (10), ein Behälter (20), eine Ausgabeeinrichtung (30), wie eine Pumpe oder ein Ventil, die auf dem Behälter (20) montiert ist, ein einstückig mit dem Körper (10) ausgebildetes seitliches Betätigungssystem (40), wobei die Vorrichtung einen Ring (50) aufweist, der auf dem Behälter (20) montiert oder einstückig mit diesem ausgebildet ist, wobei der Ring (50) mindestens einen radialen Vorsprung (55) aufweist, der dafür geeignet ist, mit einem beweglichen Element (45) des seitlichen Betätigungssystems (40) zusammenzuwirken, **dadurch gekennzeichnet, dass** der mindestens eine Vorsprung (55) mit einer geneigten radialen Endfläche (56) abgeschrägt ist.

2. Vorrichtung nach Anspruch 1, wobei der Ring (50) zwei koaxiale und symmetrische radiale Vorsprünge (55) aufweist, die sich jeweils zu beiden Seiten des Rings (50) erstrecken.

3. Vorrichtung nach Anspruch 1 oder 2, wobei der mindestens eine Vorsprung (55) einen runden, insbesondere kreisrunden Querschnitt aufweist.

4. Vorrichtung nach einem der vorhergehenden Ansprüche, wobei das bewegliche Element (45) des seitlichen Betätigungssystems (40) eine Öse (46) für jeden radialen Vorsprung (55) des Rings (50) aufweist, wobei die Öse (46) den radialen Vorsprung (55) umgibt.

5. Vorrichtung nach Anspruch 4, wobei die Öse (46) eine runde, insbesondere längliche, Innenkontur aufweist.

6. Vorrichtung nach einem der vorhergehenden Ansprüche, wobei die Ausgabeeinrichtung (30) eine Pumpe ist, die einen Kolben (31) aufweist, der in einem Pumpenkörper (32) gleitet, wobei der Ring (50) eine axiale Hülse (500) aufweist, von welcher ein erstes axiales Ende (51) einen Mantel bildet, der die Ruheposition des Kolbens (31) definiert, und von welcher ein zweites axiales Ende (520) auf einem rohrförmigen Führungsteil (530) verwirklicht ist, das dazu geeignet ist, den Kolben (31) beim Gleiten zu führen.

7. Vorrichtung nach Anspruch 6, wobei eine Druckvorrichtung auf der Pumpe (30) angebracht ist, wobei die Druckvorrichtung einen Teil aufweist, der im Inneren des zweiten axialen Endes (520) angebracht wird.

8. Vorrichtung nach Anspruch 7, wobei sich die axiale Hülse (530) bis zu dem axialen Ende des Kolbens (31) erstreckt.

9. Vorrichtung nach Anspruch 7 oder 8, wobei das zweite axiale Ende (520) abgeschrägt ist, um die Anbringung der Druckvorrichtung zu erleichtern.

10. Vorrichtung nach einem der vorhergehenden Ansprüche, wobei der Ring (50) der Befestigungsring der Ausgabeeinrichtung (30) auf dem Behälter (20) ist.

## Claims

1. A fluid dispenser device comprising: a body (10); a reservoir (20); a dispenser member (30), such as a pump or a valve, that is mounted on the reservoir (20); and a lateral actuator system (40) that is secured to said body (10); the device further comprising a ring (50) that is mounted on or secured to the reservoir (20), said ring (50) including at least one radial projection (55) that is adapted to co-operate with a movable element (45) of said lateral actuator system (40), the device being **characterized in that** said at least one projection (55) is beveled with a sloping radial end surface (56).

2. A device according to claim 1, wherein said ring (50) includes two radial projections (55) that are on a common axis and symmetrical, respectively extending on either side of said ring (50).

3. A device according to claim 1 or claim 2, wherein said at least one projection (55) has a section that is round, in particular circular.

4. A device according to any preceding claim, wherein said movable element (45) of said lateral actuator system (40) includes an eyelet (46) for each radial projection (55) of the ring (50), said eyelet (46) surrounding said radial projection (55).

5. A device according to claim 4, wherein said eyelet (46) has an inner outline that is round, in particular oblong.

6. A device according to any preceding claim, wherein the dispenser member (30) is a pump including a piston (31) that slides in a pump body (32), said ring (50) including an axial sleeve (500) having a first axial end (510) that forms a ferrule defining the rest position of the piston (31), and having a second axial end (520) that is formed on a tubular guide portion (530) that is adapted to guide the piston (31) while it is sliding.

7. A device according to claim 6, wherein a pusher is assembled on said pump (30), said pusher including a portion that comes to be assembled inside said second axial end (520).

8. A device according to claim 7, wherein said axial sleeve (530) extends as far as the axial end of said piston (31).

9. A device according to claim 7 or claim 8, wherein the second axial end (520) is beveled so as to make it easier to assemble said pusher.

10. A device according to any preceding claim, wherein said ring (50) is the fastener ring for fastening said dispenser member (30) on said reservoir (20).
